# EUROPÄISCHE PATENTSCHRIFT

(11) **EP 3 228 239 B1**
(45) Veröffentlichungstag und Bekanntmachung des Hinweises auf die Patenterteilung: **06.02.2019**
(21) Anmeldenummer: 16164101.4
(22) Anmeldetag: 06.04.2016
(51) Int. Cl.: A61B 5/00, G01N 13/00, G01N 15/08, C12M 1/12, G01N 33/483

(54) **VORRICHTUNG UND VERFAHREN ZUR MESSUNG EINER DURCH DIFFUSION DURCH MINDESTENS EIN PROBENMATERIAL ABGEGEBENEN MENGE EINES STOFFES FÜR EIN MESSZELLENSYSTEM**
DEVICE AND METHOD FOR MEASURING A QUANTITY OF A SUBSTANCE BY DIFFUSION THROUGH AT LEAST ONE SAMPLE MATERIAL FOR A MEASURING CELL SYSTEM
DISPOSITIF ET PROCEDE DE MESURE D'UNE QUANTITE DE SUBSTANCE EVACUEE PAR DIFFUSION A TRAVERS AU MOINS UN MATERIAU D'ECHANTILLON POUR UN SYSTEME DE CAPTEUR

(43) Veröffentlichungstag der Anmeldung: 11.10.2017
(73) Patentinhaber: Courage + Khazaka electronic GmbH, 50829 Köln (DE)
(72) Erfinder: Khazaka, Gabriel, 50829 Köln (DE); Hoss, Michael, 50829 Köln (DE)
(74) Vertreter: dompatent von Kreisler Selting Werner - Partnerschaft von Patent- und Rechtsanwälten mbB

(56) Entgegenhaltungen:
- EP-A1- 2 966 432
- WO-A1-94/28111
- WO-A1-2011/067587
- US-A1- 2013 210 131

## Beschreibung

Die Erfindung betrifft eine Vorrichtung zur Messung einer durch Diffusion durch mindestens ein Probenmaterial abgegebenen Menge eines Stoffes für ein Messzellensystem nach dem Oberbegriff des Anspruchs 1, sowie ein Verfahren zur Messung einer durch Diffusion durch mindestens ein Probenmaterial abgegebenen Menge eines Stoffes für ein Messzellensystem nach dem Oberbegriff des Anspruchs 14.

In der kosmetischen Industrie werden zunehmend Tests mit bzw. an in vitro hergestellten Probenmaterial, insbesondere Zellkulturen durchgeführt. In vitro bedeutet organische Vorgänge, die außerhalb eines lebenden Organismus stattfinden. Für kosmetische Tests müssen Messungen an dem in vitro hergestellten Probenmaterial durchgeführt werden. Dieses Probenmaterial ist häufig in Messzellensystemen angeordnet. Solche Messzellensysteme weisen mindestens eine, vorzugsweise mehrere Messzellen auf, die jeweils einen ersten Behälter, in dem jeweils das Probenmaterial angeordnet ist, und einen zweiten Behälter aufweisen, wobei der erste Behälter in dem jeweiligen zweiten Behälter angeordnet ist. Für die Messungen werden Messköpfe mit mindestens einer Messeinrichtung verwendet, wobei die Messköpfe auf das Messzellensystem aufgesetzt werden. Solche Messköpf sind aus der in vivo Messung, z.B. von Messungen auf der Haut bekannt. In vivo Messungen bezeichnet Messungen, die an einem lebendigen Organismus, wie beispielsweise der menschlichen Haut, durchgeführt werden Es besteht jedoch das Problem, dass die Messungen an dem Probenmaterial in dem Messzellensystem und den in vivo Messungen z.B. auf der Haut nicht vergleichbar sind.

Der Erfindung liegt daher die Aufgabe zugrunde, eine Vorrichtung und Verfahren zur Messung einer durch Diffusion durch mindestens ein Probenmaterial abgegebenen Menge eines Stoffes für ein Messzellensystem zu schaffen, bei der die Messungen in dem Messzellensystem mit in vivo Messungen vergleichbar sind.

Zur Lösung dieser Aufgabe dienen die Merkmale der Ansprüche 1 und 14.

Die Erfindung sieht in vorteilhafter Weise vor, dass die Messeinrichtung mindestens eine erste und eine zweite Sensoreinrichtung aufweist, wobei die erste und die zweite Sensoreinrichtung in einem definiertem Abstand zueinander angeordnet sind, wobei die Geometrie und die Abmessungen des Messkopfes derart an zumindest eine Messzelle des Messzellensystems angepasst ist, dass der Messkopf derart auf dem ersten Behälter platzierbar ist, dass die Sensoreinrichtungen jeweils einen definierten Abstand zu dem Probenmaterial und/oder zu einer im ersten Behälter angeordneten Referenzebene aufweisen.

Die Erfindung hat den Vorteil, dass der Messkopf an die Messzelle des Messzellensystems angepasst ist und die Sensoreinrichtungen einen definierten Abstand zu dem Probenmaterial und/oder einer im ersten Behälter angeordneten Referenzebene platzierbar ist. Dadurch die Die Messung des Messkopfes so einstellbar, dass die Messung mit in vivo Messungen vergleichbar sind.

Es können mehrere Messköpfe vorgesehen sind, die ein Messköpfesystem bilden, wobei die jeweiligen Messköpfe jeweils mindestens eine Messeinrichtung mit jeweils mindestens einer ersten und einer zweiten Sensoreinrichtung aufweisen, wobei die jeweilige erste und zweite Sensoreinrichtung in einem definiertem Abstand zueinander angeordnet sind, wobei die Messköpfe sowohl einen definierten Abstand zueinander als auch eine definierte Geometrie und Abmessung aufweisen, so dass jede der Messeinrichtung jeweils in eine Messzelle eines Messzellensystems einführbar ist und die jeweiligen Sensoreinrichtungen des jeweiligen Messkopfes jeweils einen definierten Abstand zu dem Probenmaterial und/oder zu einer im ersten Behälter angeordneten Referenzebene aufweist.

Der definierte Abstand zwischen den Sensoreinrichtungen und dem Probenmaterial und/oder der in dem ersten Behälter angeordneten Referenzebene gemäß der vorliegenden Erfindung ist vorzugsweise ein reproduzierbarer Abstand. Der definierte Abstand zwischen den Sensoreinrichtungen und dem Probenmaterial und/oder der in dem ersten Behälter angeordneten Referenzebene kann vorzugsweise zwischen 2 und 20mm liegen. Der definierte Abstand zwischen der ersten Sensoreinrichtung und dem Probenmaterial und/oder der in dem ersten Behälter angeordneten Referenzebene kann vorzugsweise zwischen 2 und 10 mm, vorzugsweise 4 mm betragen. Der definierte Abstand zwischen der zweiten Sensoreinrichtung und dem Probenmaterial und/oder der in dem ersten Behälter angeordneten Referenzebene kann vorzugsweise zwischen 8 und 20 mm, vorzugsweise 10 mm betragen. Der definierte Abstand zwischen der ersten Sensoreinrichtung und der zweiten Sensoreinrichtung kann vorzugsweise zwischen 2 und 12 mm, vorzugsweise 6 mm betragen.

Dies hat den Vorteil, dass viele Messungen in dem Messzellensystem gleichzeitig durchgeführt werden können.

Das Messprinzip der Messeinrichtung kann vorzugsweise auf den Fick'schen Diffusionsgesetz beruhen. Die erste und die zweite Sensoreinrichtung können jeweils einen vordefinierten Abstand zueinander jedoch vorzugsweise unterschiedlichen Abstand von der Oberfläche des Probenmaterials aufweisen. Mittels der ersten und der zweiten Sensoreinrichtung können die Temperatur und die relative Luftfeuchtigkeit gemessen werden. Die durch Diffusion durch das Probenmaterial abgegebene Menge eines Stoffs kann vorzugsweise verdunstetes Wasser sein. Der Partialdruck des Wasserdampfes kann von der ersten und der zweiten Sensoreinrichtung gemessen werden. Das Gefälle des mittels der ersten und der zweiten Sensoreinrichtung gemessenen Partialdrucks kann zum Verdunstungsgrad direkt proportional sein, so dass die Wasserdurchlässigkeit des Probenmaterials bestimmt werden kann.

Der mindestens eine Messkopf kann in Richtung des Probenmaterials und in die von dem Probenmaterial abgewandte Richtung offen sein.

Der mindestens eine Messkopf kann einen Messraum aufweisen, der eine erste und eine zweite Öffnung aufweist, wobei die Messeinrichtung in dem Messraum angeordnet sein kann und wobei die erste Öffnung über dem Probenmaterial platzierbar ist.

Der Messraum kann kaminförmig, insbesondere zylinderkreisförmig ausgebildet sein.

Der Messraum kann durch den ersten Behälter gebildet sein.

Die mindestens eine Messeinrichtung kann ein plattenförmiges Element aufweisen, auf dem die erste und die zweite Sensoreinrichtung angeordnet sind.

Der mindestens eine Messkopf kann eine Verbindungseinrichtung aufweisen, mit der der mindestens eine Messkopf formschlüssig und lösbar mit dem Messzellensystem verbindbar ist.

Der mindestens eine Messkopf kann auf der Messzelle und/oder auf dem Messzellensystems abgestützt sein. Die Geometrie und die Abmessungen des Messkopfes ist vorzugsweise derart an zumindest eine Messzelle des Messzellensystems angepasst ist, dass der Messkopf derart auf der Messzelle und/oder auf dem Messzellensystems abgestützt ist, dass die Sensoreinrichtungen jeweils in einen definierten Abstand zu dem Probenmaterial und/oder zu einer im ersten Behälter angeordneten Referenzebene platzierbar sind. Die Abstützung kann auf einer Fläche der Messzelle oder auf einer Fläche des Messzellensystems erfolgen. Eine Fläche der Messzelle kann eine Fläche des ersten und/oder zweiten Behälters sein. Eine Fläche des Messzellensystems kann die Oberseite des Messzellensystems sein.

Der mindestens eine Messkopf kann eine Messfläche aufweisen, wobei die Messeinrichtung bezogen auf diese Messfläche die durch Diffusion durch das mindestens eine Probenmaterial abgegebene Menge eines Stoffes misst, wobei die Geometrie und die Abmessungen des Messkopfes derart an zumindest eine Messzelle des Messzellensystems angepasst ist, dass die Messfläche konzentrisch und vorzugsweise deckungsgleich zu dem mindestens einen Probenmaterial anordnenbar ist.

Ein Zwischenadapter kann vorgesehen sein, der an den ersten Behälter derart angepasst ist, dass der erste Behälter mittels des Zwischenadapters fixierbar ist, so dass der Messkopf in einer definierten Position zu dem ersten Behälter positionierbar ist.

Es kann eine Speicher- und/oder Verarbeitungseinrichtung vorgesehen sein, wobei die mindestens eine Messeinrichtung des Messkopfes und die Speicher- und/oder Verarbeitungseinrichtung miteinander verbunden sind, so dass die Messdaten der Messeinrichtung in der Speicherung- und/oder Verarbeitungseinrichtung gespeichert und/oder verarbeitet werden können.

Es kann gemäß der vorliegenden Erfindung ein System aus einer Vorrichtung zur Messung einer durch Diffusion durch mindestens ein Probenmaterial abgegebenen Menge eines Stoffes nach einem der Ansprüche 1 bis 11 und einem Messzellensystem vorgesehen sein, wobei das mindestens eine Probenmaterial in dem Messzellensystem angeordnet ist, wobei das Messzellensystem mindestens eine, vorzugsweise mehrere Messzellen aufweist, die jeweils einen ersten Behälter, in dem jeweils ein Probenmaterial angeordnet ist, und einen zweiten Behälter aufweisen, wobei der erste Behälter in dem jeweiligen zweiten Behälter angeordnet ist.

Die Verbindungseinrichtung kann die Vorrichtung zur Messung einer durch Diffusion durch mindestens ein Probenmaterial abgegebenen Menge eines Stoffes und das Messzellensystem miteinander lösbar verbinden.

Es kann gemäß der vorliegenden Erfindung ein Verfahren zur Messung einer durch Diffusion durch mindestens ein Probenmaterial abgegebenen Menge eines Stoffes für ein Messzellensystem vorgesehen sein, wobei das mindestens eine Probenmaterial in einem Messzellensystem angeordnet ist, wobei das Messzellensystem mindestens eine, vorzugsweise mehrere Messzellen aufweist, die jeweils einen ersten Behälter, in dem jeweils ein Probenmaterial angeordnet ist, und einen zweiten Behälter aufweisen, wobei der erste Behälter in dem jeweiligen zweiten Behälter angeordnet ist, wobei folgender Verfahrensschritt vorgesehen ist
- Bereitstellen mindestens eines Messkopfes, der mindestens eine Messeinrichtung mit mindestens einer ersten und eine zweite Sensoreinrichtung aufweist, wobei die erste und die zweite Sensoreinrichtung in einem definiertem Abstand zueinander angeordnet werden, wobei die Geometrie und die Abmessungen des Messkopfes derart an zumindest eine der Messzellen des Messzellensystems angepasst werden, dass der Messkopf derart auf dem ersten Behälter platziert wird, dass die Sensoreinrichtungen in einem definierten Abstand zu dem Probenmaterial und/oder zu einer im ersten Behälter angeordneten Referenzebene platziert werden kann.

Der bereitgestellte mindestens eine Messkopf kann mit zumindest einer Messzelle des Messzellensystems verbunden werden, so dass der Messkopf derart auf dem ersten Behälter platziert wird, dass die Sensoreinrichtungen in einem definierten Abstand zu dem Probenmaterial und/oder zu einer im ersten Behälter angeordneten Referenzebene platziert werden können und die Messung mit der Messeinrichtung durchgeführt wird.

Das Probenmaterial können Zellkulturen sein. Diese können auf einer Membran angeordnet sein, die in dem ersten Behälter eingesetzt sein kann. Die Referenzebene kann eine beliebige Ebene innerhalb des ersten Behälters sein. Diese kann die Unterseite oder Oberseite des ersten Behälters bilden. Alternativ kann diese Referenzebene durch die Membran verlaufen auf der das Probenmaterial angeordnet ist. Die Referenzebene verläuft vorzugsweise parallel zu dem Probenmaterial.

Das Messzellensystem kann verschiedene Abmessungen aufweisen. Auch kann das Messzellensystem mindestens 1, mindestens 6, mindestens 12, mindestens 16, mindestens 20, mindestens 24, mindestens 48 oder mindestens 96 Messzellen aufweisen. Die Geometrie und die Abmessungen des mindestens einen Messkopfes bzw. des Messköpfeystems ist vorzugsweise jeweils an ein bestimmtes Messzellensystem angepasst. Aufgrund der Vielzahl von verschiedenen auf dem Markt erhältlichen Messzellensystemen können auch verschiedene Messköpfe bzw. Messköpfesystem bereitgestellt werden.

Im Folgenden werden unter Bezugnahme auf die Zeichnungen Ausführungsbeispiele der Erfindung näher erläutert.

Es zeigen schematisch:
- Fig. 1: Vorrichtung zur Messung einer durch Diffusion durch mindestens ein Probenmaterial abgegebenen Menge eines Stoffes für ein Messzellensystem
- Fig. 2: die Vorrichtung aus Fig. 1 in der geschnittenen Seitenansicht,
- Fig. 3: eine alternative Vorrichtung zur Messung einer durch Diffusion durch mindestens ein Probenmaterial abgegebenen Menge eines Stoffes für ein Messzellensystem,
- Fig. 4: eine weitere alternative erfindungsgemäße Vorrichtung in der geschnittenen Seitenansicht,
- Fig. 5: eine weitere alternative erfindungsgemäße Vorrichtung in der geschnittenen Seitenansicht,

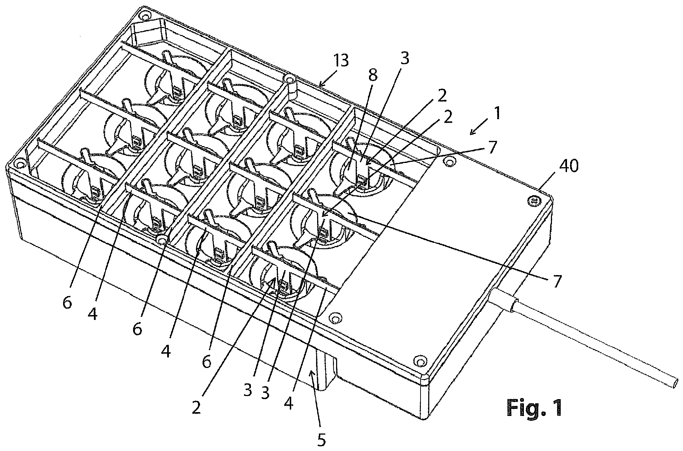

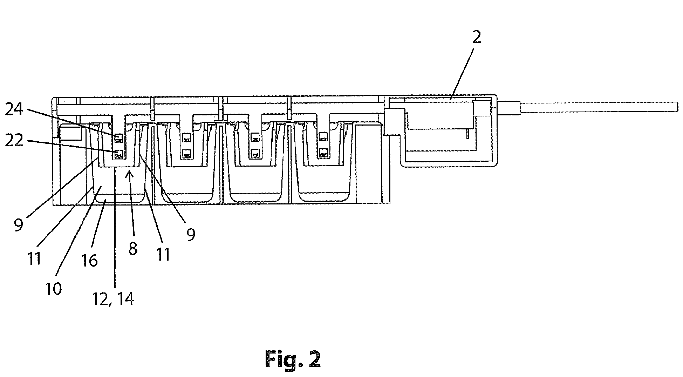

Fign. 1 und 2 zeigt eine Vorrichtung 1 zur Messung einer durch Diffusion durch mindestens ein Probenmaterial 12 abgegebenen Menge eines Stoffes für ein Messzellensystem 5. Die Fig. 2 ist die geschnittene Seitenansicht der Fig. 1. In Fig. 2 wurde auf die Schraffur der geschnittenen Teile verzichtet.

Das Probenmaterial 12 ist in dem Messzellensystem 5 angeordnet. Das Messzellensystem 5 weist mindestens eine, vorzugsweise mehrere Messzellen 7 auf. Die Messzellen 7 weisen jeweils einen ersten Behälter 8 und eine zweiten Behälter 10 auf. In dem ersten Behälter 8 ist jeweils ein Probenmaterial 12 angeordnet. Das Probenmaterial 12 können Zellkulturen sein. Das Probenmaterial 12 ist vorzugsweise auf einer Membran 14 angeordnet, die in dem ersten Behälter 8 angeordnet ist. Das Probenmaterial 12 und die Membran 14 verlaufen vorzugsweise im Wesentlichen parallel zueinander. Das Probenmaterial kann, wie im dargestellten Ausführungsbeispiel, am Boden des ersten Behälters 8 angeordnet sein. Der Behälter 8 ist am Boden lediglich durch das Probenmaterial und die Membran 14 verschlossen. Ohne Probenmaterial 12 und Membran 14 würde der Behälter 8 an der Oberseite und am Boden offen sein.

Der erste Behälter 8 ist in dem zweiten Behälter 10 angeordnet. Die Wandungen 9 des ersten Behälters 8 sind vorzugsweise mit Abstand von den Wandungen 11 des zweiten Behälters 10 angeordnet. In dem zweiten Behälter 10 kann eine Nährflüssigkeit 16 für das Probenmaterial 12 angeordnet sein.

Wie ferner Fign 1 und 2 entnommen werden kann, ist mindestens ein Messkopf 2 vorgesehen, der eine Messeinrichtung 3 aufweist. Im dargestellten Ausführungsbeispiel sind mehrere Messköpfe 2 vorgesehen, die ein Messköpfesystem 13 bilden. Die jeweilige Messeinrichtung 3 der jeweiligen Messköpfe 2 weist jeweils mindestens eine erste und eine zweite Sensoreinrichtung 22, 24 auf. Die erste und die zweite Sensoreinrichtung 22, 24 weisen einen definierten bzw. vorbestimmten Abstand zueinander auf. Die Geometrie und die Abmessungen des zumindest einen Messkopfes 2 ist derart an zumindest eine Messzelle 7 des Messzellensystems 5 angepasst ist, dass der jeweilige Messkopf 2 derart auf dem ersten Behälter 8 platzierbar ist, dass die Sensoreinrichtungen 22, 24 jeweils einen definierten Abstand zu dem Probenmaterial 12 und/oder zu einer im ersten Behälter 8 angeordneten Referenzebene platzierbar ist. Die erste und die zweite Sensoreinrichtung 22,24 weisen jeweils vorzugsweise einen unterschiedlichen Abstand zu dem Probenmaterial 12 bzw. der Referenzebene auf. Die Referenzebene kann beispielsweise durch die Membran 14 verlaufen. Die Referenzebene kann jedoch auch jede beliebige Ebene sein, die durch den ersten Behälter verläuft.

Im dargestellten Ausführungsbeispiel weist der jeweilige Messkopf 2 eine Sützeinrichtung 30 auf, die jeweils Längs- und Querstreben 4, 6 aufweist, wobei die Querstreben 6 der Stützeinrichtungen 30 der jeweiligen benachbarten Messköpfe 2 ineinander übergehen. Ferner weist der jeweilige Messkopf 2 das plattenförmige Element 26 auf, auf dem die Messeinrichtung 3 mit der ersten und zweiten Sensoreinrichtung 22, 24 angeordnet ist. Die Sützeinrichtungen 30 und die plattenförmigen Elemente 26 und somit die Messköpfe 2 sind derart ausgebildet, dass die Messköpfe 2 auf dem Messzellensystem 5 abgestützt sind und die plattenförmigen Element 26 der Messköpfe 2 mit den Sensoreinrichtungen 22, 24 in den ersten Behälter 8 hineinragen, so dass die Sensoreinrichtungen 22,24 einen definierten Abstand zu dem Probenmaterial und/oder zu einer im ersten Behälter 8 angeordneten Referenzebene aufweisen. Der jeweilige Messkopf 2 ist somit derart auf dem ersten Behälter 8 platzierbar ist, dass die Sensoreinrichtungen 22, 24 jeweils einen definierten Abstand zu dem Probenmaterial 12 und/oder zu einer im ersten Behälter 8 angeordneten Referenzebene platzierbar sind.

In den Fign. 1 und 2 ist ferner eine Speicher- und/oder Verarbeitungseinrichtung 40 vorgesehen, wobei die mindestens eine Messeinrichtung 3 des Messkopfes 2 und die Speicher- und/oder Verarbeitungseinrichtung 40 miteinander verbunden sind, so dass die Messdaten der Messeinrichtung 3 in der Speicherung- und/oder Verarbeitungseinrichtung gespeichert und oder verarbeitet werden können.

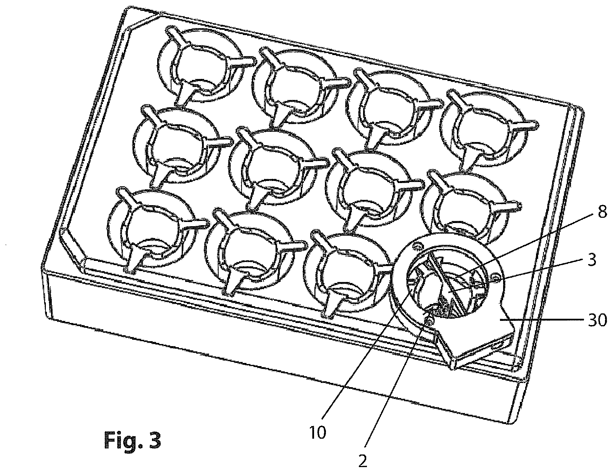

In Fig. 3 ist ein alternatives Ausführungsbeispiel dargestellt. Es ist das gleiche Messzellensystem wie in den Fign. 1 und 2. Das Ausführungsbeispiel unterscheidet sich jedoch von den Fign. 1 und 2 dadurch, dass lediglich ein Messkopf 2 vorgesehen ist. Der Messkopf 2 ist jedoch ähnlich aufgebaut wie die einzelnen Messköpfe 2 des Messköpfesystems 13. Der in Fig. 3 dargestellt Messkopf 2 weist eine Sützeinrichtung 31 auf, die jedoch im Gegensatz zu der Stützeinrichtung 30 ringförmig um die Messzelle 7 angeordnet ist. Der Messkopf 2 ist somit auf der Messzelle und/oder auf dem Messzellensystem 5 abgestützt. Das plattenförmige Element 26 ist derart gewählt, dass die Sensoreinrichtungen 22,24 einen definierten Abstand zu dem Probenmaterial und/oder zu einer im ersten Behälter 8 angeordneten Referenzebene aufweisen.

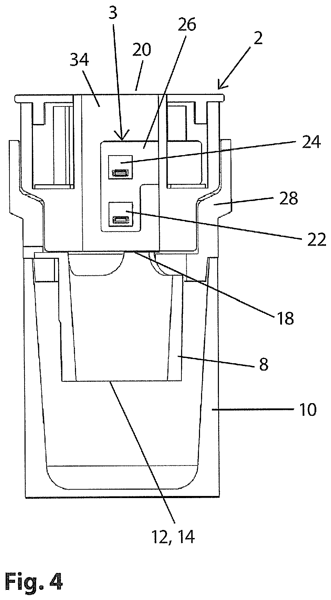

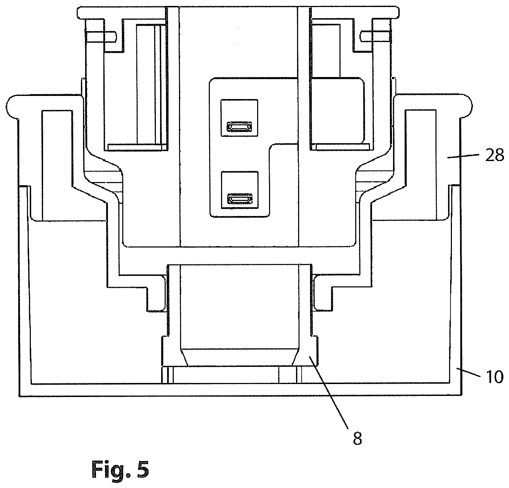

In den Fign. 4 und 5 sind erfindungsgemäße Vorrichtungen 1 dargestellt. Die Vorrichtungen 1 sind jeweils geschnitten dargestellt, wobei auf die Schraffur bei den geschnittenen Teilen verzichtet worden ist.

Die Vorrichtungen 1 weisen einen Messkopf 2 auf. Der in den Fig. 4 und 5 dargestellte Messkopf 2 weist einen Messraum 34 auf, der eine erste und eine zweite Öffnung 18, 20 aufweist, wobei die erste Öffnung oberhalb des Probenmaterials 12 angeordnet ist.

Das Messzellensystem kann, wie in den Fign. 4 und 5 dargestellt ist, lediglich eine Messzelle aufweisen.

In den Fign. 4 und 5 ist ein Zwischenadapter 28 vorgesehen, der an den ersten Behälter 8 derart angepasst ist, dass der erste Behälter 8 mittels des Zwischenadapters 28 fixierbar ist, so dass der Messkopf in einer definierten Position zu dem ersten Behälter positionierbar ist.

## Patentansprüche

1. Vorrichtung zur Messung einer durch Diffusion durch mindestens ein Probenmaterial abgegebenen Menge eines Stoffes für ein Messzellensystem, wobei das mindestens eine Probenmaterial in einem Messzellensystem angeordnet ist, wobei das Messzellensystem mindestens eine, vorzugsweise mehrere Messzellen aufweist, die jeweils einen ersten Behälter, in dem jeweils ein Probenmaterial angeordnet ist, und einen zweiten Behälter aufweisen, wobei der erste Behälter in dem jeweiligen zweiten Behälter angeordnet ist, mit
- mindestens einem Messkopf, der mindestens eine Messeinrichtung aufweist,
**dadurch gekennzeichnet,**
**dass** die Messeinrichtung mindestens eine erste und eine zweite Sensoreinrichtung aufweist, wobei die erste und die zweite Sensoreinrichtung in einem definiertem Abstand zueinander angeordnet sind, wobei die Geometrie und die Abmessungen des Messkopfes derart an zumindest eine Messzelle des Messzellensystems angepasst ist, dass der Messkopf derart auf dem ersten Behälter platzierbar ist, dass die Sensoreinrichtungen jeweils in einem definierten Abstand zu dem Probenmaterial und/oder zu einer im ersten Behälter angeordneten Referenzebene platzierbar sind.

2. Vorrichtung nach Anspruch 1, **dadurch gekennzeichnet, dass** mehrere Messköpfe vorgesehen sind, die ein Messköpfesystem bilden, wobei die jeweiligen Messköpfe jeweils mindestens eine Messeinrichtung mit jeweils mindestens einer ersten und einer zweiten Sensoreinrichtung aufweisen, wobei die jeweilige erste und zweite Sensoreinrichtung in einem definiertem Abstand zueinander angeordnet sind, wobei die Messköpfe sowohl einen definierten Abstand zueinander als auch eine definierte Geometrie und Abmessung aufweisen, so dass jede der Messeinrichtung jeweils in eine Messzelle eines Messzellensystems einführbar ist und die jeweiligen Sensoreinrichtungen des jeweiligen Messkopfes jeweils einen definierten Abstand zu dem Probenmaterial und/oder zu einer im ersten Behälter angeordneten Referenzebene aufweist.

3. Vorrichtung nach Anspruch 1 oder 2, **dadurch gekennzeichnet, dass** der mindestens eine Messkopf in Richtung Probenmaterial und in die von dem Probenmaterial abgewandte Richtung offen ist.

4. Vorrichtung nach einem der Ansprüche 1 bis 3, **dadurch gekennzeichnet, dass** der mindestens eine Messkopf einen Messraum aufweist, der eine erste und eine zweite Öffnung aufweist, wobei die Messeinrichtung in dem Messraum angeordnet ist und wobei die erste Öffnung über dem Probenmaterial platzierbar ist.

5. Vorrichtung nach Anspruch 4, **dadurch gekennzeichnet, dass** der Messraum durch den ersten Behälter gebildet ist.

6. Vorrichtung nach einem der Ansprüche 1 bis 5, **dadurch gekennzeichnet, dass** die mindestens eine Messeinrichtung ein plattenförmiges Element aufweist, auf dem die erste und die zweite Sensoreinrichtung angeordnet sind.

7. Vorrichtung nach einem der Ansprüche 1 bis 6, **dadurch gekennzeichnet, dass** der mindestens eine Messkopf eine Verbindungseinrichtung aufweist, mit der der mindestens eine Messkopf formschlüssig und lösbar mit dem Messzellensystem verbindbar ist.

8. Vorrichtung nach einem der Ansprüche 1 bis 7, **dadurch gekennzeichnet, dass** der mindestens eine Messkopf auf der Messzelle und/oder auf dem Messzellensystems abgestützt ist.

9. Vorrichtung nach einem der Ansprüche 1 bis 8, **dadurch gekennzeichnet, dass** der mindestens eine Messkopf eine Messfläche aufweist, wobei die Messeinrichtung bezogen auf diese Messfläche die durch Diffusion durch das mindestens eine Probenmaterial abgegebene Menge eines Stoffes misst, wobei die Geometrie und die Abmessungen des Messkopfes derart an zumindest eine Messzelle des Messzellensystems angepasst ist, dass die Messfläche konzentrisch und vorzugsweise deckungsgleich zu dem mindestens einen Probenmaterial anordnenbar ist.

10. Vorrichtung nach einem der Ansprüche 1 bis 9, **dadurch gekennzeichnet, dass** ein Zwischenadapter vorgesehen ist, der an den ersten Behälter derart angepasst ist, dass der erste Behälter mittels des Zwischenadapters fixierbar ist, so dass der Messkopf in einer definierten Position zu dem ersten Behälter positionierbar ist.

11. Vorrichtung nach einem der Ansprüche 1 bis 10, **dadurch gekennzeichnet, dass** eine Speicher- und/oder Verarbeitungseinrichtung vorgesehen sind, wobei die mindestens eine Messeinrichtung des Messkopfes und die Speicher- und/oder Verarbeitungseinrichtung miteinander verbunden sind, so dass die Messdaten der Messeinrichtung in der Speicherung- und/oder Verarbeitungseinrichtung gespeichert und oder verarbeitet werden können.

12. System einer Vorrichtung zur Messung einer durch Diffusion durch mindestens ein Probenmaterial abgegebenen Menge eines Stoffes nach einem der Ansprüche 1 bis 11 und ein Messzellensystem, wobei das mindestens eine Probenmaterial in dem Messzellensystem angeordnet ist, wobei das Messzellensystem mindestens eine, vorzugsweise mehrere Messzellen aufweist, die jeweils einen ersten Behälter, in dem jeweils ein Probenmaterial angeordnet ist, und einen zweiten Behälter aufweisen, wobei der erste Behälter in dem jeweiligen zweiten Behälter angeordnet ist.

13. System nach Anspruch 12, **dadurch gekennzeichnet, dass** die Verbindungseinrichtung die Vorrichtung zur Messung einer durch Diffusion durch mindestens ein Probenmaterial abgegebenen Menge eines Stoffes und das Messzellensystem miteinander lösbar verbindet.

14. Verfahren zur Messung einer durch Diffusion durch mindestens ein Probenmaterial abgegebenen Menge eines Stoffes, wobei das mindestens eine Probenmaterial in einem Messzellensystem angeordnet ist, wobei das Messzellensystem mehrere Messzellen aufweist, die jeweils einen ersten Behälter, in dem jeweils ein Probenmaterial angeordnet ist, und einen zweiten Behälter aufweisen, wobei der erste Behälter in dem jeweiligen zweiten Behälter angeordnet ist, durch
- Bereitstellen mindestens eines Messkopfes der mindestens eine Messeinrichtung mit mindestens einer ersten und eine zweite Sensoreinrichtung aufweist, wobei die erste und die zweite Sensoreinrichtung in einem definiertem Abstand zueinander angeordnet werden, wobei die Geometrie und die Abmessungen des Messkopfes derart an zumindest eine der Messzellen des Messzellensystems angepasst werden, dass der Messkopf derart auf dem ersten Behälter platziert wird, dass die Sensoreinrichtungen in einem definierten Abstand zu dem Probenmaterial und/oder zu einer im ersten Behälter angeordneten Referenzebene platziert werden kann.

15. Verfahren nach Anspruch 14, **dadurch gekennzeichnet, dass** der bereitgestellte mindestens eine Messkopf mit zumindest einer Messzelle des Messzellensystems verbunden wird, so dass der Messkopf derart auf dem ersten Behälter platziert wird, dass die Sensoreinrichtungen in einem definierten Abstand zu dem Probenmaterial und/oder zu einer im ersten Behälter angeordneten Referenzebene platziert werden kann. und die Messung mit der Messeinrichtung durchgeführt wird.

## Claims

1. Device for measuring a quantity of a substance given off by diffusion through at least one sample material for a measuring cell system, wherein the at least one sample material is arranged in a measuring cell system, wherein the measuring cell system comprises at least one, preferably a plurality of measuring cells, each comprising a first container in which a sample material is arranged, respectively, and a second container, wherein the first container is arranged in the respective second container, comprising
- at least one measuring head comprising at least one measuring means,
**characterized in that**
the measuring means comprises at least one first and one second sensor means, wherein the first and the second sensor means are arranged at a defined mutual distance, wherein the geometry and the dimensions of the measuring head are adapted to at least one measuring cell of the measuring cell system such that the measuring head can be placed on the first container such that the sensor means can each be placed at a defined distance from the sample material and/or from a reference plane arranged in the first container.

2. Device of claim 1, **characterized in that** a plurality of measuring heads is provided which form a measuring head system, wherein the respective measuring heads each comprise at least one measuring means with at least one first and one second sensor means, wherein the respective first and second measuring means are arranged at a defined mutual distance, wherein the measuring heads both have a defined mutual distance and a defined geometry and dimensions, so that each of the measuring means is adapted to be respectively inserted into a measuring cell of a measuring cell system and the respective sensor means of the respective measuring head each have a defined distance from the sample material and/or from a reference plane arranged in the first container.

3. Device of claim 1 or 2, **characterized in that** the at least one measuring head is open to the sample material and in the direction averted from the sample material.

4. Device of one of claims 1 to 3, **characterized in that** the at least one measuring head comprises a measuring space having a first and a second opening, wherein the measuring means is arranged in the measuring space, and wherein the first opening is adapted to be placed above the sample material.

5. Device of claim 4, **characterized in that** the measuring space is formed by the first container.

6. Device of one of claims 1 to 5, **characterized in that** at least one measuring means comprises a plate-shaped element on which the first and the second sensor means are arranged.

7. Device of one of claims 1 to 6, **characterized in that** the at least one measuring head comprises a connecting means by which the at least one measuring head can be connected with the measuring cell system in a positive and detachable manner.

8. Device of one of claims 1 to 7, **characterized in that** the at least one measuring head is supported on the measuring cell and/or on the measuring cell system.

9. Device of one of claims 1 to 8, **characterized in that** the at least one measuring head comprises a measuring surface, wherein the measuring means measures - with respect to the measuring surface - the quantity of a substance given off by diffusion through at least one sample material, wherein the geometry and the dimensions of the measuring head are adapted to at least one measuring cell of the measuring cell system such that the measuring surface is adapted to be arranged concentrically and preferably congruently with the at least one sample material.

10. Device of one of claims 1 to 9, **characterized in that** an intermediate adapter is provided adapted to the first container such that the first container can be fixed by means of the intermediate adapter, so that the measuring head can be positioned in a defined position relative to the first container.

11. Device of one of claims 1 to 10, **characterized in that** a memory and/or a processing device are provided, wherein the at least one measuring means of the measuring head and the memory and/or processing means are connected with each other, so that the measuring data of the measuring means can be stored and/or processed in the memory and/or processing means.

12. System of a device for measuring a quantity of a substance given off by diffusion through at least one sample material as defined in one of claims 1 to 11, and a measuring cell system, wherein the at least one sample material is arranged in the measuring cell system, wherein the measuring cell system comprises at least one, preferably a plurality of measuring cells, each comprising a first container in which a sample material is arranged, respectively, and a second container, wherein the first container is arranged in the respective second container.

13. System of claim 12, **characterized in that** the connecting means detachably connects the device for measuring a quantity of a substance given off by diffusion through at least one sample material and the measuring cell system.

14. Method for measuring a quantity of a substance given off by diffusion through at least one sample material, wherein he at least one sample material is arranged in the measuring cell system, wherein the measuring cell system comprises at least one, preferably a plurality of measuring cells, each comprising a first container in which a sample material is arranged, respectively, and a second container, wherein the first container is arranged in the respective second container, the method comprising the step of
- providing at least one measuring head comprising at least one measuring means with at least one first and one second sensor means, wherein the first and the second sensor means are arranged at a defined mutual distance, wherein the geometry and the dimensions of the measuring head are adapted to at least one measuring cell of the measuring cell system such that the measuring head is placed on the first container such that the sensor means can each be placed at a defined distance from the sample material and/or from a reference plane arranged in the first container.

15. Method of claim 14, **characterized in that** the at least one measuring head provided is connected with at least one measuring cell of the measuring cell system, so that the measuring head is placed on the first container such that the sensor means that the sensor means can each be placed at a defined distance from the sample material and/or from a reference plane arranged in the first container and the measuring is performed using the measuring means.

## Revendications

1. Appareil de mesure d'une quantité d'une substance distribuée par diffusion à travers au moins un échantillon pour un système de cellule de mesure, dans lequel l'au moins un échantillon est agencé dans un système de cellule de mesure, dans lequel le système de cellule de mesure comporte au moins une, de préférence plusieurs cellules de mesure comportant chacune un premier récipient à l'intérieur duquel est respectivement agencé un échantillon et un deuxième récipient, dans lequel le premier récipient est agencé à l'intérieur du deuxième récipient respectif, doté
- d'au moins une tête de mesure, comportant au moins un dispositif de mesure,
**caractérisé en ce que** le dispositif de mesure comporte au moins un premier et un deuxième dispositif de capteur, dans lequel le premier et le deuxième dispositif de capteur sont agencés avec un écart défini l'un par rapport à l'autre, dans lequel la géométrie et les dimensions de la tête de mesure sont adaptées à au moins une cellule de mesure du système de cellule de mesure de telle sorte que la tête de mesure peut être placée sur le premier récipient de telle façon que les dispositifs de capteur peuvent être placés respectivement avec un écart défini par rapport à l'échantillon et/ou par rapport à un plan de référence agencé dans le premier récipient.

2. Appareil selon la revendication 1, **caractérisé en ce que** plusieurs têtes de mesure sont prévues, lesquelles constituent un système de têtes de mesure, dans lequel chaque tête de mesure comporte respectivement au moins un dispositif de mesure avec respectivement au moins un premier et un deuxième dispositif de capteur, dans lequel les dispositifs de capteur respectifs sont agencés avec un écart défini l'un par rapport à l'autre, dans lequel les têtes de mesure comportent aussi bien un écart défini l'une par rapport à l'autre qu'une géométrie et des dimensions définies, de sorte que chacun des dispositifs de mesure peut être introduit respectivement dans une cellule de mesure d'un système de cellule de mesure et les dispositifs de capteur respectifs de la tête de mesure respective comportent respectivement un écart défini par rapport à l'échantillon et/ou par rapport à un plan de référence agencé dans le premier récipient.

3. Appareil selon la revendication 1 ou 2, **caractérisé en ce que** l'au moins une tête de mesure est ouverte dans la direction de l'échantillon et dans la direction se détournant de l'échantillon.

4. Appareil selon l'une des revendications 1 à 3, **caractérisé en ce que** l'au moins une tête de mesure comporte un espace de mesure, comportant une première et une deuxième ouverture, dans lequel le dispositif de mesure est agencé dans l'espace de mesure et dans lequel la première ouverture peut être placée au-dessus de l'échantillon.

5. Appareil selon la revendication 4, **caractérisé en ce que** l'espace de mesure est formé par le biais du premier récipient.

6. Appareil selon l'une des revendications 1 à 5, **caractérisé en ce que** l'au moins un dispositif de mesure comporte un élément en forme de plaque, sur lequel sont agencés le premier et le deuxième dispositifs de capteur.

7. Appareil selon l'une des revendications 1 à 6, **caractérisé en ce que** l'au moins une tête de mesure comporte un dispositif de raccordement, au moyen duquel l'au moins une tête de mesure peut être raccordée au système de cellule de mesure par complémentarité de forme et de manière détachable.

8. Appareil selon l'une des revendications 1 à 7, **caractérisé en ce que** l'au moins une tête de mesure repose sur la cellule de mesure et/ou sur le système de cellule de mesure.

9. Appareil selon l'une des revendications 1 à 8, **caractérisé en ce que** l'au moins une tête de mesure comporte une surface de mesure, dans lequel le dispositif mesure par rapport à cette surface la quantité d'une substance distribuée par diffusion à travers au moins un échantillon, dans lequel la géométrie et les dimensions de la tête de mesure sont adaptées à au moins une cellule de mesure du système de cellule de mesure de sorte que la surface de mesure peut être agencée de manière concentrique et de préférence en concordance avec l'au moins un échantillon.

10. Appareil selon l'une des revendications 1 à 9, **caractérisé en ce qu'**un adaptateur intermédiaire est prévu, lequel est ajusté au premier récipient de sorte que le premier récipient peut être fixé au moyen d'un adaptateur intermédiaire, de façon à ce que la tête de mesure puisse être positionnée dans une position définie par rapport au premier récipient.

11. Appareil selon l'une des revendications 1 à 10, **caractérisé en ce qu'**un dispositif d'enregistrement et/ou de traitement est prévu, dans lequel l'au moins un dispositif de mesure de la tête de mesure et le dispositif d'enregistrement et/ou de traitement sont raccordés l'un à l'autre, de sorte que les données de mesure du dispositif de mesure peuvent être enregistrées et/ou traitées dans le dispositif d'enregistrement et/ou de traitement.

12. Système d'un appareil de mesure d'une quantité d'une substance distribuée par diffusion à travers au moins un échantillon selon l'une des revendications 1 à 11 et d'un système de cellule de mesure, dans lequel l'au moins un échantillon est agencé dans le système de cellule de mesure, dans lequel le système de cellule de mesure comporte au moins une, de préférence plusieurs cellules de mesure comportant chacune un premier récipient à l'intérieur duquel est respectivement agencé un échantillon et un deuxième récipient, dans lequel le premier récipient est agencé à l'intérieur du deuxième récipient respectif.

13. Système selon la revendication 12, **caractérisé en ce que** le dispositif de raccordement raccorde l'un à l'autre de manière détachable l'appareil de mesure d'une quantité d'une substance distribuée par diffusion à travers au moins un échantillon et le système de cellule de mesure.

14. Procédé de mesure d'une quantité d'une substance distribuée par diffusion à travers au moins un échantillon, dans lequel l'au moins un échantillon est agencé dans un système de cellule de mesure, dans lequel le système de cellule de mesure comporte plusieurs cellules de mesure, comportant chacune un premier récipient, à l'intérieur duquel un échantillon est respectivement agencé, et un deuxième récipient, dans lequel le premier récipient est agencé à l'intérieur du second récipient, par
- préparation d'au moins une tête de mesure de l'au moins un dispositif de mesure comportant au moins un premier et un deuxième dispositif de capteur, dans lequel le premier et le deuxième dispositif de capteur sont agencés l'un par rapport à l'autre avec un écart défini, dans lequel la géométrie et les dimensions de la tête de mesure sont ajustées à au moins une des cellules de mesure du système de cellule de mesure de telle sorte que la tête de mesure est placée sur le premier récipient de telle sorte que les dispositifs de capteur peuvent être placés à une distance définie par rapport à l'échantillon et/ou par rapport à un plan de référence agencé dans le premier récipient.

15. Procédé selon la revendication 14, **caractérisé en ce que** l'au moins une tête de mesure préparée est raccordée à au moins une cellule de mesure du système de cellule de mesure, de sorte que la tête de mesure est placée sur le premier récipient de façon à ce que les dispositifs de capteur puissent être placés à une distance définie par rapport à l'échantillon et/ou par rapport à un plan de référence agencé dans le premier récipient, et **en ce que** la mesure est effectuée avec le dispositif de mesure.
